# EUROPEAN PATENT SPECIFICATION

(11) **EP 3 402 911 B1**
(45) Date of publication and mention of the grant of the patent: **25.08.2021**
(21) Application number: 17702780.2
(22) Date of filing: 13.01.2017
(51) Int. Cl.: C25B 3/00, C07C 51/41, C25B 15/08, C25B 1/02, C25B 1/04, B01D 61/00, C07C 51/00, C07C 59/08, C07C 53/02, C07C 51/16, C12P 7/56

(54) **PROCESS FOR PRODUCING OXALIC ACID**
VERFAHREN ZUR HERSTELLUNG VON OXALSÄURE
PROCÉDÉ DE PRODUCTION D'ACIDE OXALIQUE

(30) Priority: 13.01.2016 US 201662278282 P
(43) Date of publication of application: 21.11.2018
(73) Proprietor: Avantium Knowledge Centre B.V., 1014 BV Amsterdam (NL)
(72) Inventor: KACZUR, Jerry J., North Miami Beach Florida 33160 (US); LAKKARAJU, Prasad P., East Brunswick New Jersey 08816 (US); PARAJULI, Rishi R., Kendell Park New Jersey 08824 (US)
(74) Representative: Dijkhuis ev Boon, Anouk Hélène J.
(86) International application number: PCT/EP2017/050735
(87) International publication number: WO 2017/121887

(56) References cited:
- EP-A2- 0 329 337
- WO-A1-2014/046791
- WO-A1-2015/184388
- WO-A1-2015/195149
- US-A- 5 723 049
- LESTER G LUNDSTED: "The Hydrogenation of Sodium Bicarbonate to Sodium Formate", JOURNAL OF THE AMERICAN CHEMICAL SOCIETY, vol. 71, no. 1, 1 January 1949 (1949-01-01), pages 323-324, XP055349338,
- MANUEL CHECA ET AL: "Catalytic transformation of glycerol on several metal systems supported on ZnO", CATALYSIS TODAY, vol. 196, no. 1, 15 March 2012 (2012-03-15), pages 91-100, XP55089869, ISSN: 0920-5861, DOI: 10.1016/j.cattod.2012.02.036
- KANG, U. ET AL: "Photosynthesis of formate from CO2 and water at 1% energy efficiency via copper iron oxide catalysis", ENERGY & ENVIRONMENTAL SCIENCE, vol. 8, 24 June 2015 (2015-06-24), pages 2638-2543, XP002771019,

## Description

### FIELD OF THE INVENTION

The present invention relates to processes for producing formate and/or formic acid, and lactic acid.

### BACKGROUND TO THE INVENTION

The combustion of fossil fuels in activities such as the electricity generation, transportation, and manufacturing produces billions of tons of carbon dioxide annually. Research since the 1970s indicates increasing concentrations of carbon dioxide in the atmosphere may be responsible for altering the Earth's climate, changing the pH of the ocean, and other potentially damaging effects. Countries around the world, including the United States, may be seeking ways to mitigate emissions of carbon dioxide.

One implementation may be to convert carbon dioxide into economically valuable materials such as fuels and industrial chemicals. If the carbon dioxide may be converted using energy from renewable sources, it will be possible to both mitigate carbon dioxide emissions and to convert renewable energy into a chemical form that may be stored for later use. Electrochemical and photochemical pathways may be likely mechanisms for carbon dioxide conversion.

WO2015/184388 describes a process for reducing carbon dioxide comprising: receiving a feed of hydrogen gas at an anolyte region of an electrochemical cell including an anode; receiving an anolyte feed at the anolyte region of the electrochemical cell; receiving a catholyte feed including carbon dioxide and an alkali metal bicarbonate at a catholyte region of the electrochemical cell including a cathode; applying an electrical potential between the anode and the cathode of the electrochemical cell sufficient to reduce the carbon dioxide to a reduction product, wherein the anolyte feed to the electrochemical cell includes water and a hydrogen halide and wherein the reduction product is alkali metal formate. WO2015/184388 further describes that this alkali metal formate can be converted into an alkali metal oxalate via a thermal reaction and that the alkali metal oxalate can be converted to oxalic acid in an electrochemical acidification electrolyzer. Manuel Checa et al., "Catalytic transformation of glycerol on several metal systems support on ZnO", Catalysis Today, vol. 196, no. 1, 15 March 20212, pages 91-100 discloses a process for the catalytic transformation of glycerol to yield lactic acid on several metal systems supported on ZnO. Kang, U et al., "Photosynthesis of formate from CO2 and water at 1 % energy efficiency via copper iron oxide catalysis", Energy & Environmental Science, vol. 8, 24 June 2015, pages 2638-2543, discloses a process for the preparation of formate from carbon dioxide and water via copper iron oxide catalysis.

Although good results are obtained with the process and/or system of WO2015/184388, there is still room for alternative processes and further improvement.

It would therefore be an advancement in the art to have a process with which similar, better, or economically more advantageous results can be achieved.

### SUMMARY OF THE INVENTION

Such processes have been achieved with the process according to the invention as defined by the appending claims.

Accordingly, the present invention provides a process for producing formic acid and/or formate from CO₂ and lactic acid from glycerol, comprising:
- a stage 1, wherein a metal M is reacted with carbon dioxide and water to thereby produce a metal oxide of metal M and formic acid and/or formate; and
- a stage 2, wherein the metal oxide of metal M is reacted with glycerol to thereby produce metal, lactic acid and water,
wherein the metal M is iron, zinc, aluminium or manganese.

Disclosed herein is further a process for producing oxalic acid containing or having the following steps:
i) Utilizing a chemical reaction to produce an alkali metal formate and/or alkaline earth metal formate;
ii) Converting the alkali metal formate and/or alkaline earth metal formate to an alkali metal oxalate and/or alkaline earth metal oxalate in a thermal reaction, preferably utilizing hydrogen in the process;
iii) Converting the alkali metal oxalate and/or alkaline earth metal oxalate to oxalic acid and an alkali metal base and/or alkaline earth metal base utilizing an electrochemical process; and
iv) Recycling the alkali metal base and/or alkaline earth metal base from step (iii) to step (i).

Preferably step (i) of such a process comprises or consists of utilizing a chemical reaction to convert carbon, carbon monoxide, carbon dioxide, methane, hydrogen or a combination of any of these to thereby produce an alkali metal formate and/or alkaline earth metal formate.

The present disclosure thus provides a process and system for a combined chemical and electrochemical process for the production of oxalic acid from carbon dioxide that can be subsequently converted to a downstream product such as monoethylene glycol (MEG). That is, the current disclosure also provide a process for the production of MEG, wherein an oxalic acid obtained or obtainable by a process as described herein is converted to MEG. In a first step, the process may utilize Syngas, carbon monoxide produced from carbon or carbon dioxide, and/or hydrogen as a feedstock for the first process step where alkali metal carbonate and/or alkali metal bicarbonate is converted to an alkali metal formate. In a second step, the alkali metal formate may then be reacted in a thermal reactor to produce an alkali metal oxalate intermediate product. In a third step, an electrochemical process step may then be used to convert the alkali metal oxalate to oxalic acid and an alkali metal hydroxide. The alkali metal hydroxide may then be reacted with carbon dioxide to form the alkali metal carbonate and/or alkali metal bicarbonate utilized in the first process step formation of alkali metal formate. The process may utilize carbon dioxide and raw materials obtained from biological sources as well as electrical power from renewable sources.

### BRIEF DESCRIPTION OF THE DRAWINGS

The processes of the invention are illustrated by the accompanying figures in which:
Figure 1 illustrates an example of an integrated thermal and electrochemical process using syngas to produce formate. It illustrates the use of syngas in a series flow through two separate CO and H₂ formate reactors.
Figure 2 illustrates an example of an integrated thermal and electrochemical process using hydrogen (H₂) to produce formate.
Figure 3 illustrates an example of an integrated thermal and electrochemical process using carbon monoxide (CO) to produce formate.
Figure 4 shows alternative routes to formate using hydrogen
Figure 5 shows alternative routes to formate using carbon monoxide.

### DETAILED DESCRIPTION OF THE INVENTION

The present disclosure describes a process and system for a combined chemical and electrochemical process for the production of oxalic acid from carbon dioxide that can be subsequently converted to a downstream product such as monoethylene glycol (MEG).

Disclosed herein is an alternative process for producing oxalic acid having the following process steps:
- In a first step, utilizing of one or more chemical route reactions in producing alkali metal formate from a carbon, carbon dioxide, or methane and their combinations
- In a second step, converting of the alkali metal formate to an alkali metal oxalate in a thermal reaction and utilizing any co-products, such as hydrogen, in the process
- In a third step, converting the alkali metal oxalate to oxalic acid and an alkali metal base utilizing various electrochemical process
- In a fourth step, recycling the alkali metal base from the electrochemical system back into the formate chemical route as needed in addition to any other suitable process co-products such as hydrogen, oxygen, or chlorine depending on the electrochemical cell anode and cathode reactions and its chemical feed(s)

That is, the process is preferably a process, wherein the alkali metal base and/or alkaline earth metal base in step (iv) is recycled from step (iii) into step (i) in addition to hydrogen, oxygen, or chlorine.

It is contemplated that the process may employ full or partial use of the electrochemical formate cells for the reduction of carbon dioxide to formate. That is, preferably the process is a process wherein step (i) further includes the use of an electrochemical cell for the reduction of carbon dioxide to formate.

In addition, the process may preferably consume more CO₂ than may be produced using the process feed options available. The process raw materials may be chosen such that the entire process may be operated on a commercial scale and may have suitable economics. The formation and use of alkali metal formate may be preferred, but the formation and use of alkaline earth formates using reagents such as calcium hydroxide or calcium carbonate, may also be employed, so long as a good process of separation and recycle of the alkaline earth metals with minimal generation of wastes may be designed. Hence, hereafter, wherever an alkali metal compound is described, an alkaline earth metal compound, although less preferred, may also be used.

### Chemical Reactants for Formate Formation

Various chemical feed stocks may be suitable in the chemical formation of alkali metal formate. The chemical reactions with proposed and theorized chemical reactions are summarized below.

### CO Reactions

Carbon monoxide (CO) is a very reactive reagent that is useful in carbonylation reactions.
a. CO Reaction with Alkali Metal Hydroxide, for example:

   CO + KOH → KCO₂H

   This is a reaction for producing alkali metal formate. The CO source may be from various sources such as Syngas, RWGS reaction, and the like. The reaction operating temperature may range from 50°C to 300°C at pressures between 445 and 13,890 kPa (50 to 2,000 psig). Process to increase the reaction rate may employ packed bed towers with counter flow of CO as well as employing reactive spray drying equipment. Catalysts may be optionally employed and the reaction may employ an aqueous or an aqueous system with an added solvent, such as an alcohol, to help improve the reaction rate. The solvent would be recovered and recycled in a separate step. Solid state reactions with a wetted alkali metal hydroxide may also be suitable with a sufficient mixing/reaction system.
   That is, step (i) of the process according to the disclosure may comprise or consist of reacting carbon monoxide with an alkali metal hydroxide and/or an alkaline earth metal hydroxide to thereby produce the corresponding alkali metal formate and/or alkaline earth metal formate.
b. CO Reaction with Alkali Metal Carbonate, for example:

   K₂CO₃ + 2CO + H₂O → 2KCO₂H + CO₂

   The reaction operating temperature for this reaction may range from 100°C to 300°C at pressures between 445 and 13,890 kPa (50 to 2,000 psig). Process of increasing the reaction rate may employ packed bed towers with counter flow of CO as well as employing reactive spray drying equipment. Catalysts may be optionally employed and the reaction may employ an aqueous or an aqueous system with an added solvent, such as an alcohol, to help improve the reaction rate. The solvent would be recovered and recycled in a separate step. Solid state reactions with a wetted alkali metal carbonate may also be suitable with a sufficient mixing/reaction system.
   That is, step (i) of the process according to the disclosure may comprise or consist of reacting carbon monoxide with an alkali metal carbonate and/or an alkaline earth metal carbonate and with water to thereby produce the corresponding alkali metal formate and/or alkaline earth metal formate and carbon dioxide.
c. CO Reaction with Alkali Metal Bicarbonate (also referred to as Alkali Metal Hydrogen Carbonate), for example:

   KHCO₃ + CO → KCO₂H + CO₂

   This reaction may or may not require a catalyst. If both CO and hydrogen are used as the reactant gas (a water gas composition), the reaction may occur with or without a nickel catalyst which may help with the reaction with hydrogen, and then both gases may react to form formate, and requiring less CO in the reaction. The reaction operating temperature may range from 100°C to 350°C at pressures between 445 and 34,574 kPa (50 to 5,000 psig). Catalysts may be optionally employed and the reaction may employ an aqueous or an aqueous system with an added solvent, such as an alcohol to help improve the reaction rate. The solvent would be recovered and recycled in a separate step. Solid state reactions with wetted alkali metal bicarbonate may be suitable with a sufficient mixing/reaction system.
   That is, step (i) of the process according to the disclosure may comprise or consist of reacting carbon monoxide with an alkali metal bicarbonate and/or an alkaline earth metal bicarbonate to thereby produce the corresponding alkali metal formate and/or alkaline earth metal formate and carbon dioxide.
d. Formaldehyde (gas) Reaction with Alkali Metal Hydroxide

   HCHO(g) + KOH → KCO₂H + H₂

   HCHO(g) → CH₃OH(aq) Side reaction

   This reaction operating temperature may range from 20°C to 150°C at pressures between 101 and 3537 kPa (0.1 and 500 psig). The reaction of formaldehyde forming methanol is a side reaction that may be minimized using lower reaction temperatures. Catalysts may be optionally employed and the reaction may employ an aqueous or an aqueous system with an added solvent, such as an alcohol to help improve the reaction rate. The system may also be totally non-aqueous using one or more solvents. The solvent would be recovered and recycled in a separate step.
   That is, step (i) of the process according to the disclosure may comprise or consist of reacting a, preferably gaseous, formaldehyde with an alkali metal hydroxide and/or an alkaline earth metal hydroxide to thereby produce the corresponding alkali metal formate and/or alkaline earth metal formate and hydrogen, optionally including a side reaction wherein the, preferably gaseous, formaldehyde is converted to methanol.
e. CO Reaction with Steam to Make Formic Acid and Neutralize with Alkali Metal Hydroxide
   This reaction requires a high temperature and pressure in addition to a catalyst in the formation of the formic acid. The reaction operating temperature may range from 100°C to 350°C at pressures between 445 and 34,574 kPa (50 to 5,000 psig). Catalysts such as the phosphates of zinc, calcium, magnesium, sodium, potassium, copper, and cerium as well as the acid salts of these formed with the oxides of tungsten and molybdenum and mixtures.

   Reaction 1 CO + H₂O → HCO₂H (with Catalyst)

   Reaction 2 HCO₂H + KOH → KCO₂H + H₂O

   That is, step (i) of the process according to the disclosure may comprise or consist of reacting carbon monoxide with steam to make formic acid and subsequently neutralize the formic acid with an alkali metal hydroxide and/or alkaline earth metal hydroxide to thereby produce the corresponding alkali metal formate and/or alkaline earth metal formate.
f. Alkali Metal Carbonate with Hydrogen

   K₂CO₃ + 2H₂ → 2KCO₂H + H₂O

   This reaction operating temperature may range from 100°C to 300°C at pressures between 445 and 13,890 kPa (50 to 2,000 psig). The reaction may require a catalyst to work efficiently. The catalyst may be a homogeneous or heterogeneous type.
   That is, step (i) of the process according to the disclosure may comprise or consist of reacting an alkali metal carbonate and/or an alkaline earth metal carbonate with hydrogen to thereby produce the corresponding alkali metal formate and/or alkaline earth metal formate and water.

### Hydrogen Reactions

Hydrogen is a reactive reagent that has been used for the reduction of many chemicals.
a. Carbon Dioxide (CO₂) Reaction with Hydrogen (H₂)

   CO₂ + H₂ → HCO₂H

   The reaction operating temperature of this reaction may range from 100°C to 500°C at pressures between 445 and 34,574 kPa (50 to 5,000 psig). The reaction requires a catalyst for the reaction to work efficiently. The catalyst may be a homogeneous or heterogeneous type. The use of a base, such as carbonate, hydroxide, tertiary amine and the like helps promote the reaction.
   That is, step (i) of the process according to the disclosure may comprise or consist of reacting carbon dioxide with hydrogen to thereby produce formic acid, and optionally subsequently neutralizing the formic acid with an alkali metal hydroxide and/or alkaline earth metal hydroxide to thereby produce the corresponding alkali metal formate and/or alkaline earth metal formate.
b. Alkali Metal Carbonate Reaction with Hydrogen

   K₂CO₃ + 2H₂ → 2KCO₂H + H₂O

   As described above, the reaction operating temperature of this reaction may range from 100°C to 300°C at pressures between 445 and 13,890 kPa (50 to 2,000 psig). Catalysts may be optionally employed and the reaction may employ an aqueous or an aqueous system with an added solvent, such as an alcohol to help improve the reaction rate. The system may also be totally non-aqueous using one or more solvents. The solvent would be recovered and recycled in a separate step. The catalyst may be a homogeneous or heterogeneous type. A heterogeneous catalyst is preferred such that little or no catalyst may end up in the final product.
c. Alkali Metal Bicarbonate Reaction with Hydrogen

   KHCO₃ + H₂ → KCO₂H + H₂O

   The reaction operating temperature of this reaction may range from 100°C to 300°C at pressures between 445 and 13,890 kPa (50 to 2,000 psig). Catalysts may be optionally employed and the reaction may employ an aqueous or an aqueous system with an added solvent, such as an alcohol to help improve the reaction rate. The system may also be totally non-aqueous using one or more solvents. The solvent would be recovered and recycled in a separate step. The catalyst may be a homogeneous or heterogeneous type. A heterogeneous catalyst is preferred such that little or no catalyst may end up in the final product.
   That is, step (i) of the process according to the disclosure may comprise or consist of reacting an alkali metal bicarbonate and/or an alkaline earth metal bicarbonate with hydrogen to thereby produce the corresponding alkali metal formate and/or alkaline earth metal formate and water.

### CO and Hydrogen From Syngas and Water Gas Reactions

a. Carbon or biomass may advantageously be converted to CO utilizing CO₂ using the Boudouard reaction as follows:

   C(s) + CO₂ → 2CO
b. Carbon or biomass may be converted to CO utilizing pure O2, such as from the electrochemical cells or using PSA (Pressure Swing Absorption) to generate it from air as follows:

   C(s) + O₂ → 2CO
c. Syngas formation from methane as follows, generating both CO and H₂ which can be further processed:

   CH₄ + H₂O → CO + 3H₂
d. Reverse Water Gas Shift (RWGS) Reaction as follows, making CO from CO₂ and hydrogen:

   CO₂ + H₂ → CO + H₂O
e. Water Gas Shift Reaction (WGSR) as follows, making H2 from CO and water:

   CO + H₂O → CO₂ + H₂

That is, the carbon monoxide that may potentially be used in the process according to the disclosure may conveniently be produced or obtained by:
- a reaction of carbon or biomass with carbon dioxide;
- a reaction of carbon or biomass with oxygen;
- a reaction of methane with water; and/or
- a reaction of carbondioxide with hydrogen.

In addition, the hydrogen that may potentially be used in the process according to the disclosure may conveniently be produced or obtained by:
- a reaction of methane with water; and/or
- a reaction of carbon monoxide with water.

Step (ii) of the process according to the disclosure may conveniently comprise or consist of reacting the alkali metal formate and/or alkaline earth metal formate with molten hydroxide and carbon dioxide to thereby produce the corresponding alkali metal oxalate and/or alkaline earth metal oxalate.

Molten hydroxide may react with CO₂ and alkali metal formate to produce alkali metal oxalate. The addition of CO₂ is added as a reactant gas in a pressurized reactor and elevated temperature to produce oxalate. The reaction that may occur is the reaction of one formate, perhaps as the dianion, with one CO2 or one alkali metal carbonate to produce one oxalate. The reaction may be more effective than reacting two formates to make one oxalate.

The alkali metal formate (or formate dianion) in step (i) may also be produced in different alternative manners.

For example by a carbon dioxide (CO₂) reaction with a thermal melt. That is, one may: make a mixture of alkali metal bicarbonate or carbonate with an alkali metal hydroxide; melt the mixture; and bubble CO₂ through the mixture or react the mixture in a reactor where pressurized CO₂ is introduced into the reactor. In this embodiment, the alkali metal formate (or formate dianion) is produced in the reactor mix.

Or for example by a carbon monoxide (CO) reaction with a thermal melt. That is, one may: make a mixture of alkali metal bicarbonate or carbonate with an alkali metal hydroxide; melt the mixture; and bubble CO through the mixture or react the mixture in a reactor where pressurized CO is introduced into the reactor. The reaction may occur in a pressurized packed bed reactor. Formate may be produced which would react and convert to make alkali metal oxalate. The formate may also react with the carbonate and bicarbonate in the presence of the hydroxide to produce oxalate.

Or for example by a hydrogen reaction with a thermal melt. That is, one may: make a mixture of mixture of alkali metal bicarbonate or carbonate with an alkali metal hydroxide; melt the mixture; and bubble hydrogen through the mixture or react the mixture in a reactor where pressurized hydrogen is introduced into the reactor. The reaction may occur in a pressurized packed bed reactor. Formate may be produced which would react and convert to make alkali metal oxalate. The formate may also react with the carbonate and bicarbonate in the presence of the hydroxide to produce oxalate.

Or for example by a carbon monoxide (CO) and hydrogen reaction with a thermal melt. That is, one may: make a mixture of mixture of alkali metal bicarbonate or carbonate with an alkali metal hydroxide; melt the mixture; and bubble a mixture of CO and hydrogen through the mixture or react the mixture in a reactor where pressurized CO and hydrogen is introduced into the reactor. The reaction may occur in a pressurized packed bed reactor. Formate may be produced which would react and convert to make alkali metal oxalate. The formate may also react with the carbonate and bicarbonate in the presence of the hydroxide to produce oxalate.

All of the embodiments may utilize a catalyst, a homogeneous or heterogeneous catalyst. The reaction may also include other additives such as organics that may improve the yield of the reaction or to conceptually produce C2 and higher organic compounds from the reaction mixture. In addition, the reaction may alternatively occur in an aqueous solution mixture which may also include some amount of an organic solvent, such as an alcohol, or may be mainly a nonaqueous solution. The temperatures of the reaction may be lower, and the solvents may be recovered or may be part of the reactions to do C-C coupling.

The invention advantageously provides several routes to value added chemicals from CO₂ and bio-glycerol with non-precious metal/metal oxide redox cycles.

Hydrogenation of CO₂ will not only reduce the amount of CO₂ linked to environmental problems but also produce the useful chemicals and fuels. A carbon redox cycle that couples CO₂ hydrogenation with simultaneous oxidation of bio-based renewables (e.g. glycerol) into industrially important chemicals possess additional advantages of monetizing two wastes without requiring direct electrical energy. Thus, reducing CO₂ with biochemical reductants will have major impact on the energy landscape from a number of viewpoints and will also provide sustainable green chemicals for numerous industries.

The present invention provides a process for the production of formic acid and/or formate from CO₂ and lactic acid from glycerol.

Below indicative steps are provided for such process. These steps or stages are not intended to be limiting, but indicative only.

Indicative Step/Stage 1: CO_{2 to} Formic acid /Formate:

A hydrothermal reactor may produce (formic acid) or potassium formate by oxidizing earth abundant non-precious metal(s) as reductant(s). Metal oxide formed after the reaction may be used in a glycerol reactor and formate produced on the cathode is moved further to make MEG.

Indicative Step/Stage 2: Lactic acid production

Metal oxide from Step/Stage 1 may be used for the catalytic oxidation of glycerol to form lactic acid. Alkali metal hydroxide may be required as a catalyst for this reaction. Lactic acid can be further processed to make biopolymers.

Without wishing to be bound by any kind of theory, it is believed that indicative reactions and stoichiometry could be:
Step/Stage 1. M+CO₂+H₂O → MOₓ+ HCOOH
Step/Stage 2. MOₓ+C₃H₈O₃ →M+C₃H₆O₃+H₂O
Overall: CO₂+C₃H₈O₃ → HCOOH+C₃H₆O₃
M= Fe, Zn, Al, Mn and additional metal promotor can be used to improve reaction rate and efficiency.

Without wishing to be bound by any kind of theory it is believed that CO or carbon monoxide is a very reactive reagent that can be useful in carbonylation reactions.

There are a number of advantages which may include:
- Options for using CO₂ directly to obtain formic acid or Alkali hydroxide (e.g. KOH from EA cell) + CO₂ to obtain formate salt in step 1.
- The reducing agent for CO₂ is glycerol (or any other bio-organics that can reduce metal oxide to metal)
- KOH from the EA cell can be utilized as a catalyst
- Step 1 proceeds through quantitative H2 production from water and reacted in-situ with CO₂.

The present disclosure further provides a chemical process utilizing syngas, carbon monoxide produced from carbon dioxide and hydrogen, and/or hydrogen in first producing an alkali metal formate product. The chemical reactions may be as follows:
a. reacting of CO with an alkali metal carbonate to produce the corresponding alkali metal formate. The CO may be produced from the RWGS reaction of carbon dioxide with hydrogen. Reactions may include:
   i. CO reaction with alkali metal carbonate:

      K₂CO₃ + 2CO + H₂O → 2KCO₂H + CO₂
   ii. CO reaction with alkali metal bicarbonate

      KHCO₃ + CO → KCO₂H + CO₂
   iii. RWGS Reaction:

      CO₂ + H₂ → CO + H₂O
b. reacting of an alkali metal bicarbonate with hydrogen to produce the corresponding alkali metal formate, using a catalyst as needed. Alkali metal carbonate may also be converted to alkali metal formate, but may not be as reactive.

   KHCO₃ + H₂ → KCO₂H + H₂O

   These reactions a. and b. are preferably operated in reactions using an aqueous phase, but the aqueous solution may include other non-reactive solvents. Ionic liquids, amines, and other added components may be used in the solution to promote the reaction.
c. utilization of methane, that may be converted to syngas, which is a product mixture typically containing CO and H₂ with the basic reaction as follows using a catalyst:

   CH₄ + H₂O → CO + 3H₂

Preferably the alkali metal formate is separated from the reaction product mixtures and converted to alkali metal formate solids or concentrated solutions.

The alkali metal formate solids or concentrated solutions (used in a spray drying conversion process) can suitably be converted into alkali metal oxalates using a thermal reaction, which may also produce byproduct hydrogen that can be recovered for use in the other process steps.

The alkali metal oxalates can be dissolved in an aqueous solution and the oxalate can be converted to oxalic acid and an alkali metal hydroxide in an electrochemical process such as electrochemical acidification or electrodialysis.

The electrochemical acidification cell can produce oxalic acid, an anolyte co-product, an alkali metal hydroxide, and hydrogen from the catholyte.

The electrodialysis cell can produce oxalic acid and alkali metal hydroxide and can utilize bipolar membranes.

Conveniently the oxalic acid can then be converted to monoethylene glycol (MEG) via an esterification route followed by hydrogenation.

In one embodiment, the process may employ methane as a feedstock, which may be from a biological source, which may be converted to Syngas. The gas from the reactor may then be passed through a set of CO and H₂ based formate reactors in a series flow arrangement. The reactors may be set up with separate alkali metal carbonate and alkali metal bicarbonate feeds so that the CO in the Syngas may react with the alkali metal carbonate in a first reactor and the remaining gas, containing hydrogen and some unreacted CO, and may then be passed onto a second reactor containing alkali metal bicarbonate, with a heterogeneous catalyst as needed, to form alkali metal formate. The gases may be recycled around each reactor to enable high conversion of the Syngas components. Two CO₂ reactors may be used to provide the alkali metal carbonate and bicarbonate feed to the corresponding reactor from the alkali metal hydroxide. The alkali metal formate production of each reactor is related to the ratio of the CO to H₂ in the Syngas gas stream. The CO and H₂ alkali formate reactors may operate at pressures between 169 and 34,574 kPa (10 to 5,000 psig) and at temperatures ranging from 50°C to 500°C.

In another embodiment, biological sourced CO₂ may be converted to CO in a separate RWGS reactor to convert alkali metal carbonate to alkali metal formate. The hydrogen from the electrochemical acidification cell may be used in a separate reactor to convert alkali metal bicarbonate to alkali metal formate. The production of the alkali metal formate may depend on the quantities of hydrogen and CO available in the process.

In a further embodiment, a carbon source, such as carbon or biomass may be converted to CO utilizing CO₂ using the Boudouard reaction as follows:

C(s) + CO₂ → 2CO

In another embodiment, a carbon source, such as carbon or biomass may be converted to CO utilizing a limited amount of O₂. The oxygen source may be from the electrochemical acidification cell anolyte reaction or from pressure swing absorption oxygen enrichment units (PSA).

C(s) + O₂ → 2CO

The processes of the disclosure are further illustrated by the following nonlimiting figures.

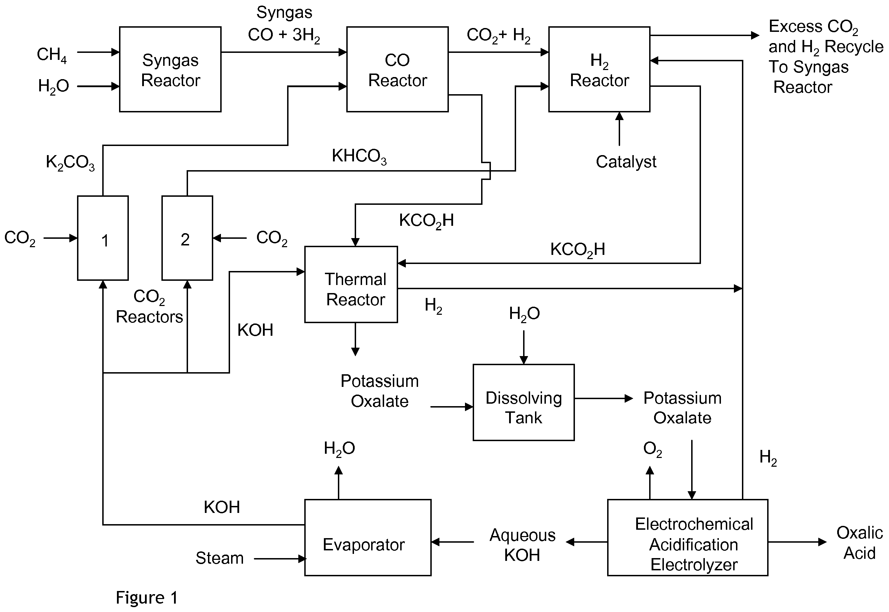

Figure 1, shows one embodiment where syngas may be produced from methane, where the methane to the Syngas reactor may be from a biological or biomass source.

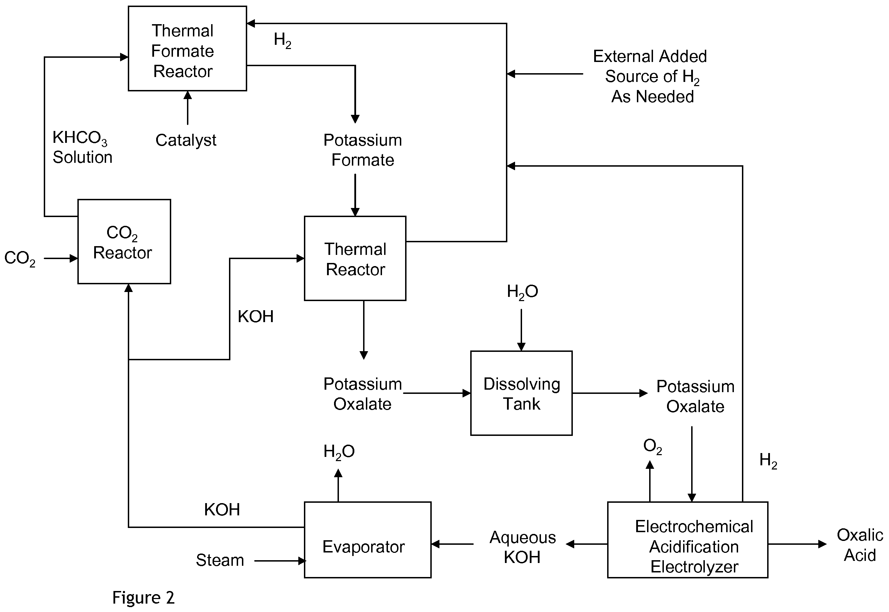

Figure 2, shows an integrated process via hydrogen.

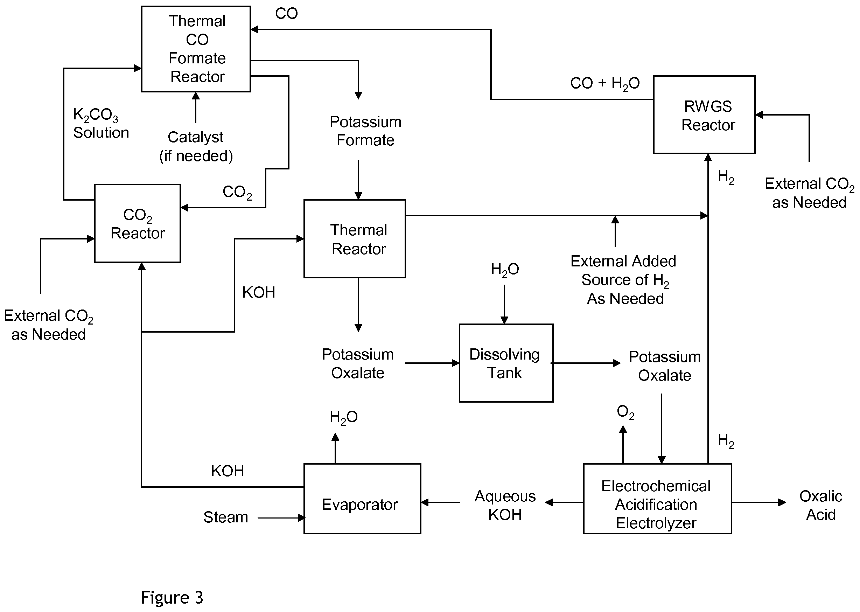

Figure 3, shows an integrated process via carbon monoxide

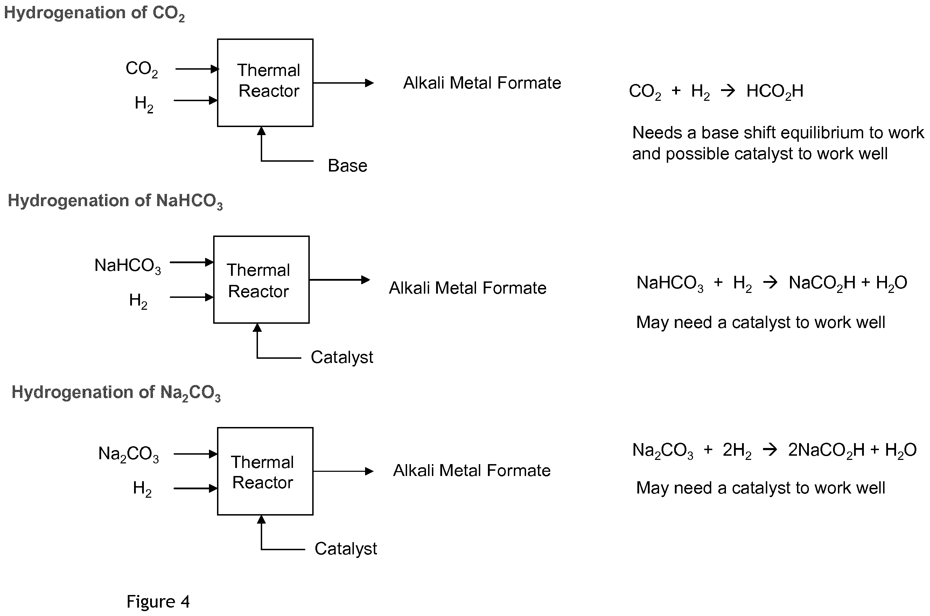

Figure 4 illustrates alternative thermal routes to formate using hydrogen

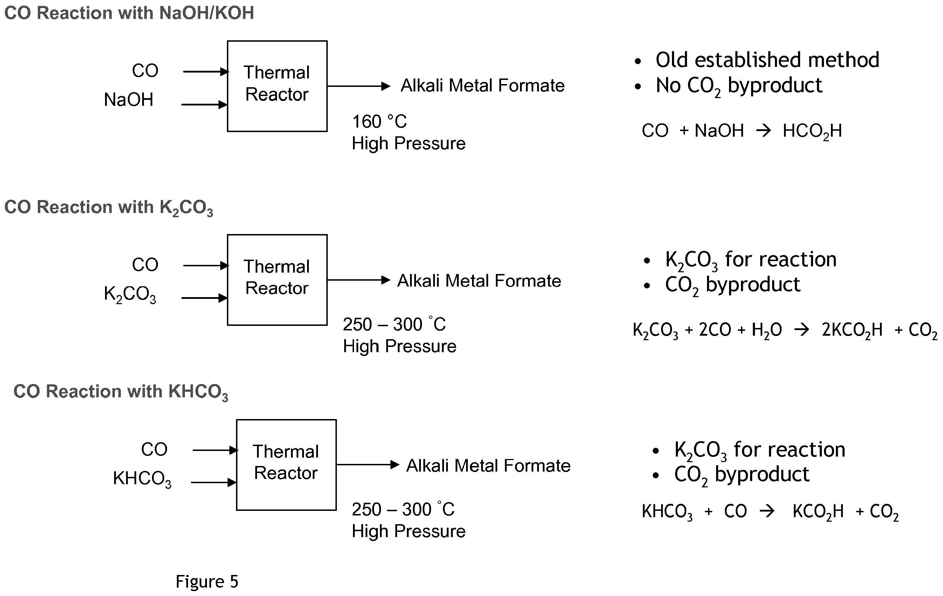

Figure 5 illustrates alternative thermal routes to formate using carbon monoxide.

As described above, carbon monoxide and hydrogen from Syngas and water-gas shift reaction may also be used.

## Claims

1. A process for the production of formic acid and/or formate from CO₂ and lactic acid from glycerol, comprising:
- a stage 1, wherein a metal M is reacted with carbon dioxide and water to thereby produce a metal oxide of metal M and formic acid and/or formate; and
- a stage 2, wherein the metal oxide of metal M is reacted with glycerol to thereby produce metal, lactic acid and water,
wherein the metal M is Iron, Zinc, Aluminium or Manganese.

2. The process according to claim 1, wherein an additional metal promotor is used.

3. The process according to claim 1 or 2, wherein stage 1 is carried out in a hydrothermal reactor.

4. The process according to any one of claims 1 to 3, wherein alkali metal hydroxide is used as a catalyst in stage 2.

5. The process according to any one of claims 1 to 4, wherein the lactic acid is further processed to make biopolymers.

6. The process according to any one of claims 1 to 5, wherein the formate is used to make monoethylene glycol.

## Patentansprüche

1. Vorgang für die Herstellung von Ameisensäure und/oder Formiat aus CO₂ und Milchsäure aus Glycerin, der Folgendes umfasst:
- eine Stufe 1, wobei ein Metall M mit Kohlendioxid und Wasser umgesetzt wird, um dadurch ein Metalloxid von Metall M und Ameisensäure und/oder Formiat herzustellen; und
- eine Stufe 2, wobei das Metalloxid von Metall M mit Glycerin umgesetzt wird, um dadurch Metall, Milchsäure und Wasser herzustellen,
wobei das Metall M Eisen, Zink, Aluminium oder Mangan ist.

2. Vorgang nach Anspruch 1, wobei ein zusätzlicher Metallpromotor verwendet wird.

3. Vorgang nach Anspruch 1 oder 2, wobei Stufe 1 in einem hydrothermalen Reaktor durchgeführt wird.

4. Vorgang nach einem der Ansprüche 1 bis 3, wobei in Stufe 2 Alkalimetallhydroxid als ein Katalysator verwendet wird.

5. Vorgang nach einem der Ansprüche 1 bis 4, wobei die Milchsäure weiterverarbeitet wird, um Biopolymere zu produzieren.

6. Vorgang nach einem der Ansprüche 1 bis 5, wobei das Formiat verwendet wird, um Monoethylenglykol zu produzieren.

## Revendications

1. Procédé de production d'acide formique et/ou de formiate à partir de CO₂ et d'acide lactique à partir de glycérol, comprenant :
- une étape 1, un métal M étant mis en réaction avec du dioxyde de carbone et de l'eau pour produire ainsi un oxyde métallique de métal M et d'acide formique et/ou de formiate ; et
- une étape 2, l'oxyde métallique de métal M étant mis en réaction avec du glycérol pour produire ainsi du métal, de l'acide lactique et de l'eau,
le métal M étant du fer, du zinc, de l'aluminium ou du manganèse.

2. Procédé selon la revendication 1, un promoteur métallique supplémentaire étant utilisé.

3. Procédé selon la revendication 1 ou 2, l'étape 1 étant effectuée dans un réacteur hydrothermal.

4. Procédé selon l'une quelconque des revendications 1 à 3, l'hydroxyde de métal alcali étant utilisé comme catalyseur à l'étape 2.

5. Procédé selon l'une quelconque des revendications 1 à 4, l'acide lactique étant en outre traité pour fabriquer des biopolymères.

6. Procédé selon l'une quelconque des revendications 1 à 5, le formiate étant utilisé pour fabriquer du monoéthylène glycol.
